# EUROPÄISCHE PATENTSCHRIFT

(11) **EP 1 385 502 B1**
(45) Veröffentlichungstag und Bekanntmachung des Hinweises auf die Patenterteilung: **14.03.2007**
(21) Anmeldenummer: 02732578.6
(22) Anmeldetag: 08.04.2002
(51) Int. Cl.: A61K 9/00, A61K 31/265, A61K 47/24, A61K 47/28, A61P 31/12, A61P 35/00, A61P 37/00

(54) **ARZNEIMITTEL ENTHALTEND EINE WASSERFREIE FORMULIERUNG EINES XANTHOGENATES**
MEDICAMENT COMPRISING AN ANHYDROUS FORMULATION OF XANTHOGENATE
MEDICAMENT COMPRENANT UNE FORMULATION ANHYDRE D'UN XANTHOGENATE

(30) Priorität: 09.04.2001 DE 10117728
(43) Veröffentlichungstag der Anmeldung: 04.02.2004
(73) Patentinhaber: Shogoo Pharmaceuticals, K.K., Chuo-ku Tokyo 103-0022 (JP)
(72) Erfinder: AMTMANN, Eberhard, 69121 Heidelberg (DE)
(74) Vertreter: Patentanwälte Zellentin & Partner
(86) Internationale Anmeldenummer: PCT/EP2002/003885
(87) Internationale Veröffentlichungsnummer: WO 2002/080901

(56) Entgegenhaltungen:
- WO-A-96/14841
- DE-A- 3 625 948
- US-A- 4 602 037

## Beschreibung

Die Erfindung betrifft Arzneimittel enthaltend eine pharmazeutische Formulierung eines Xanthogenates zur Behandlung von Virus-, Tumor- oder Autoimmunerkrankungen.

Xanthogenate, insbesondere Tricyclodecan-9yl-xanthogenat (D609) sind als Substanzen mit antiviraler und antitumoraler Aktivität bekannt, z.B. aus "DNA and RNA virus species are inhibited by xanthates, a class of antiviral compounds with unique properties" Sauer-G; Amtmann-E; Melber-K; Knapp-A; Muller-K; Hummel-K; Scherm-A, in Proc-Natl-Acad-Sci-U-S-A. 1984 Jun; 81(11): 3263-7; "Selective killing of tumor cells by xanthates" Amtmann-E; Sauer-G, in Cancer-Lett. 1987 Jun; 35(3): 237-44 und U.S. Patent No 4, 602, 037.

Die antiviralen Eigenschaften von Xanthogenaten werden jedoch gemäß "Synergistic antiviral effect of xanthates and ionic detergents" Amtmann-E; Muller-Decker-K; Hoss-A; Schalasta-G; Doppler-C; Sauer-G, in Biochem-Pharmacol. 1987 May 1; 36(9): 1545-9 nur im angesäuerten Milieu oder in Anwesenheit von ionischen Detergentien evident.

Da Xanthogenate säurelabil sind, ist ihre pharmazeutische Verwendung in angesäuerten Formulierungen aus Stabilitätsgründen nicht praktikabel. Als besonders effektive Additive haben sich die Kaliumsalze der Decan-, Undecan- und Laurinsäure erwiesen. Diese Additive weisen selber keine antiviralen Eigenschaften auf.

Jedoch sind Xanthogenate stark irritierend. Die Pharmazeutische Verwendung von Xanthogenaten war deshalb bisher nicht möglich. Für D609 wurde die maximale, tolerierbare Konzentration in Salben als auf 1% begrenzt gefunden. In Tierexperimenten wurde gezeigt, dass für eine wirkungsvolle antivirale Verwendung mindestens 2,5 % D609 in einer Salbe enthalten sein müssen.

Die pharmazeutische Verwendung von antiviral und antitumoral wirksamen Xanthogenaten stößt somit auf drei Probleme:
1. Für die pharmakologische Wirksamkeit sind relativ hohe Wirkstoffkonzentrationen notwendig. Im Tierversuch wurden zur effizienten Hemmung von Herpes simplex Virus 1 Konzentrationen von mindestens 2.5 % des Wirkstoffes benötigt.
2. Xanthogenate sind instabil im wässrigen Milieu, insbesondere bei leicht sauren Bedingungen, wie sie auf der Haut vorherrschen.
3. Xanthogenate haben ausgeprägte irritierende Eigenschaften. Die Anwendung kann deshalb nur in Formulierungen mit maximal 1 % Wirkstoff erfolgen.

Die WO 96/14841 beschreibt pharmazeutische Formulierungen, die ein Xanthogenat und ein die Aktivität des Xanthogenats erhöhendes Adjuvans in einen steroid- oder lididbasierenden Träger inkorporiert enthalten. Diese Formulierungen werden durch Ultraschallbehandlung von Suspensionen der Bestandteile in Wasser erhalten. Die Anwesenheit des Adjuvans wird als unabdingbar zur Inkorporierung des Xanthogenats beschrieben.

Nachteilig an diesen Formulierungen ist vor allem die aufwendige Herstellung. Auch enthalten die Formulierungen Wasser.

Wir haben nun überraschend gefunden, dass durch die einfache Zugabe von bestimmten Emulgatoren zu Xanthogenaten deren irritierende Wirkung überwunden werden kann. Dadurch wird die pharmazeutische Verwendung der Xanthogenate in therapeutisch wirksamen Konzentrationen möglich. Da diese Formulierungen wasserfrei sind, ist die chemische Stabilität des Wirkstoffes über längere Zeiträume gewährleistet und damit eine pharmazeutische Verwendung ermöglicht. Mit den erfindungsgemäßen Formulierungen ist es demnach überraschenderweise möglich alle drei Probleme ohne die aufwendige Inkorporierung in einen Träger zu lösen. Es wird zum einen die Verträglichkeit deutlich verbessert. Die antivirale Effizienz wird gesteigert. Auch eine ausreichende chemische Stabilität in wässrigem Milieu ist gewährleistet.

Die vorliegende Erfindung löst somit die oben genannten Probleme, indem eine pharmazeutische Formulierung bereitgestellt wird, die ein Xanthogenat, ein dessen Aktivität erhöhendes Adjuvans und ein Steroid oder Phospholipid als Emulgator enthält, der die Reizwirkung des Xanthogenats und des die Aktivität erhöhenden Adjuvans reduziert.

Die Herstellung erfolgt erfindungsgemäß so, dass das Xanthogenat und das Adjuvans in Substanz mit dem Emulgator vermengt wird, wobei bevorzugt ein nicht-wässriges Hilfsmittel, z.B. Paraffinöl, zugesetzt werden kann. Überraschenderweise ist eine Suspendierung in Wasser und/oder Ultraschallbehandlung nicht erfoderlich. Die durch einfaches Mischen, beispielsweise durch Kneten oder Rühren, erhaltenen Formulierungen zeigen die gewünschte verminderte Reizwirkung und die erforderliche Stabilität.

Die Formulierung enthält ein Xanthogenat der allgemeinen Formel 1 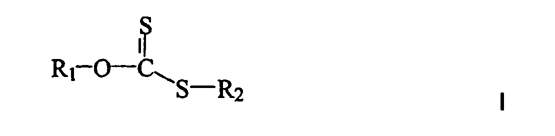 wobei R₁ für einen gegebenenfalls substituierten Aryl- oder Alkylrest steht. Vorzugsweise steht R₁ für einen Adamantyl-, Norbornyl-, Tricyclodecyl-, Benzyl-, geraden oder verzweigten C₃-C₂₀-Alkyl-, C₃-C₂₀-Cycloalkyl-, Furyl-. Pyridyl-, Anthracyl-, Naphtyl-, Phenanthryl-, Perinaphtyl-, oder Chinuclidinyl-Rest steht, und der obengenannte gerade oder verzweigte C₃-C₂₀-Alkylrest durch eine Hydroxyl-C₁-C₄-Alkoxygruppe, ein Halogenatom oder eine Aminogruppe und der obengenannte C₃-C₂₀-Cycloalkylrest ebenfalls durch eine Hydroxyl-, C₁-C₄-Alkoxy- oder C₁-C₄-Alkylgruppe, ein Halogenatom oder eine Aminogruppe substituiert sein können. Besonders vorteilhaft für R₁ sind Cyclododecyl-, Dodecyl-, Undecyl-, Decyl-, Tricyclo[5,2,1,0^{2,6}]-decyl-, nonyl-, octyl-, Bicyclo[2,2,1]-heptyl-, Cyclohexyl-, Hexyl-, Toluoyl- Reste. Ganz besonders vorteilhaft ist ein Tricyclo[5,2,1,0^{2,6}]-decylrest.

R₂ stellt ein Metallatom, eine gegebenenfalls substituierte Alkyl-, Alkoxy-, Amino- oder Ammoniumgruppe oder Halogen dar. Vorzugsweise steht R₂ für ein ein- oder mehrwertiges Metallatom, einen geraden C₁-C₆-Alkylrest, einen durch Hydroxy substituierten C₁-C₆-Alkylrest, einen C₁-C₆-Alkoxyrest, eine Aminogruppe, einen C₁-C₆-Alkaminorest, einen (C₁-C₆-Alkyl)₂-Aminorest, einen (C₁-C₆-Alkyl)₃-ammoniumrest, ein Halogen, 2,3-Dihydroxypropyl oder Hydroxy-(C₁-C₆-alkoxy)-methyl dar. Besonders vorteilhaft sind Natrium- und Kalium-Salze sowie Dimethylglycyl- und Methyl-Ester.

Das die Aktivität erhöhende Adjuvans ist vorzugsweise ein ionisches Detergens. Es kann eine Fettsäure mit 6 -19 C-Atomen oder deren Salz sein. Insbesondere vorteilhaft sind die Kaliumsalze der Decan- Undecan- oder Laurinsäure.

Das die Aktivität erhöhende Adjuvans kann auch ein Sulfat mit einem aliphatischen Rest von 8-18 C-Atomen sein. Besonders bevorzugt ist Na-Laurinsulfat.

Weiter kommt für das Adjuvans Deoxycholinsäure oder ein pharmazeutisch verträgliches Salz davon oder eine Phosphonsäure in Betracht.

Formulierungen, in denen pro ein Teil Xanthogenat 0,1 bis 10 Teile die Aktivität erhöhendes Adjuvans enthalten sind, haben sich als gut geeignet erwiesen. Besonders vorteilhaft ist ein Verhältnis Xanthogenat zu Aktivität erhöhendes Adjuvans von 1:1.

Der die Reizwirkung reduzierende Emulgator ist vorzugsweise ein Steroid. Geeignet sind beispielsweise Stearylamin, Cholesterin, Cholestanol, Cholansäure, Chondrillasterol und α, β, γ Sisterol. Besonders vorteilhaft ist Cholesterin.

Als die Reizwirkung reduzierender Emulgator eignen sich auch Phospholipide, wie Lecithin, Phosphatidylcholin, Phosphatidylserin, Phosphatidylinositol; Glycolipide wie z.B. Gangliosid, oder Sphingolipide, wie z.B. Sphingomyelin. Besonders vorteilhaft sind Lecithin, Sphingomyelin und Phosphatidylserin

Vorzugsweise werden 0,5 bis 10 Teile Emulgator je Gewichtsanteil Xanthogenat eingesetzt, insbesondere 2 bis 6 Teile.

Besonders bevorzugt ist eine Formulierung, bei der der die Reizwirkung reduzierende Emulgator Cholesterin ist, das die Aktivität erhöhende Adjuvans das Na- oder K-Salz der Decansäure ist und das Xanthogenat Tricyclo[5,2,1,0^{2,6}]-9yl-xanthogenat ist. Insbesondere kommen auf einen Teil Xanthogenat ein Teil Kaliumsalz der Decansäure und 4 Teile Cholesterin.

Eine weitere besonders bevorzugte Formulierung enthält als den Reizwirkung reduzierenden Emulgator Phosphatidylcholin, als die Aktivität erhöhendes Adjuvans das Na- oder K-Salz der Decansäure und als Xanthogenat Tricyclo[5,2,1,0^{2,6}]-9yl-xanthogenat. Insbesondere kommen auf einen Teil Xanthogenat ein Teil Decansäure und 4 Teile Phosphatidylcholin.

Weiter enthalten die Mittel übliche Hilfsstoffe. Es können auch andere Wirkstoffe enthalten sein, soweit sie weder die Wirkung noch die Stabilität der Xanthogenate beeinträchtigen.

Insbesondere bevorzugt sind Mittel in Form von Salben, wobei als Salbengrundlage eine lipophile Substanz benutzt wird. Vorzugsweise wird als Salbengrundlage Vaseline benutzt. Konzentrationen an Xanthogenat von 3 Gew.-% haben sich als Kompromiss zwischen Wirkung und unerwünschter Reizung besonders bewährt.

Eine weitere bevorzugte Ausführungsform sind wasserfreie Zubereitungen für Injektionslösungen. Diese enthalten vorzugsweise neben der erfindungsgemäßen pharmazeutischen Formulierung je Teil Xanthogenat 0,1 - 5 Teile eines Alkalihydroxids, insbesondere KOH. Das Alkalihydroxid verbessert die Löslichkeit des Adjuvans und stabilisiert zusätzlich das Xanthogenat in der Injektionslösung.

Die Injektionslösung wird für die Anwendung durch lösen bzw. emulgieren in einem physiologisch verträglichen Medium, wie z.B. isotonischer Salzlösung, erhalten. In einer besonders bevorzugten Ausführungsform wird die Xanthogenat-Emulgator-Mischung in einem Kit mit dem physiologisch verträglichen Medium bereitgestellt, wobei das Alkalihydroxid wahlweise als separate Trockensubstanz, in der Xanthogenat-Emulgator-Mischung oder in dem physiologisch verträglichen Medium gelöst vorliegt.

Die erfindungsgemäßen pharmazeutischen Formulierungen und Mittel, die sie enthalten, eignen sich zur Behandlung von Virus-, Tumor- und Autoimmunerkrankungen, wie z.B. Herpes simplex und Papillomavirusinfektionen. Die erfindungsgemäßen Salben sind besonders zur Behandlung von Hauterkrankungen geeignet.

Die folgenden Beispiele erläutern die Erfindung weiter, ohne sie jedoch zu beschränken.

### Beispiel 1

### Herstellung einer Salbe

Für 100 g Salbe werden 3 g D609 mit 12 g Cholesterin gemischt. Dabei werden zur Erleichterung der Durchmischung der pulverförmigen Bestandteile 20 - 25 g Paraffinöl zugegeben. Die Mischung wird zu einem Gesamtgewicht von 100 g mit Vaseline versetzt und das ganze in der Reibeschale mit einem Pistill gleichmäßig durchmischt. Wenn auf den Einsatz von Paraffinöl verziochtet wird, ist der Vaselineanteil entsprechend zu erhöhen.

In gleicher Weise wurde eine Salbe aus 3 g D609 und 12 g natürlichem Lecithin hergestellt.

In gleicher Weise wurde eine Salbe aus 3 g D609 und 12 g synthetischem Lecithin hergestellt.

In gleicher Weise wurde eine Salbe aus 3 g D609 und 12 g natürlichem Phosphatidylserin hergestellt.

Grössere Mengen Salbe können durch bekannte, technische Verfahren analog hergestellt werden.

### Beispiel 2

### Herstellung von Injektionslösungen

3 g D609 und 3 g Kaliumundecanat werden mit 12 g natürlichem oder synthetischem Cholesterin gemischt. Danach werden 3 g wasserfreies KOH zugegeben. Das trockene Gemisch wird unmittelbar vor der Injektion mittels isotonischer Salzlösungen gelöst bzw emulgiert. KOH kann auch separat in trockener Form oder als Lösung bereitgestellt werden oder Inhalt eines Kits sein.

In gleicher Weise wurde ein Gemisch für eine Injektionslösung hergestellt, wobei Cholesterin durch die gleiche Menge natürliches oder synthetisches Lecithin ersetzt wird.

In gleicher Weise wurde ein Gemisch für eine Injektionslösung hergestellt, wobei Cholesterin durch die gleiche Menge natürliches oder synthetisches Sphingomyelin ersetzt wird.

In gleicher Weise wurde ein Gemisch für eine Injektionslösung hergestellt, wobei Cholesterin durch die gleiche Menge natürliches oder synthetisches Phosphatidylserin ersetzt wird.

### Beispiel 3

### Irritierende Wirkung von D609 in Phosphatidylcholin- oder Cholesterin-haltigen Salben.

D609 wurde zusammen mit dem gleichen Gewichtsanteil Kaliumsalz der Decansäure und zwei Gewichtsteilen Phosphatidylcholin bzw. zwei oder vier Gewichtsteilen Cholesterin im Mörser gemischt. Danach wurden so viel dünnflüssiges Paraffinöl und Vaseline (1 Gewichtsteil Paraffinöl, 4 Teile Vaseline) zugegeben, dass jeweils eine Konzentration von 1,25; 2,5; 5,0; oder 10 % D609 erreicht war. Auf die gleiche Weise wurden D609-haltige Salben ohne Phosphatidycholin bzw. Cholesterin hergestellt.

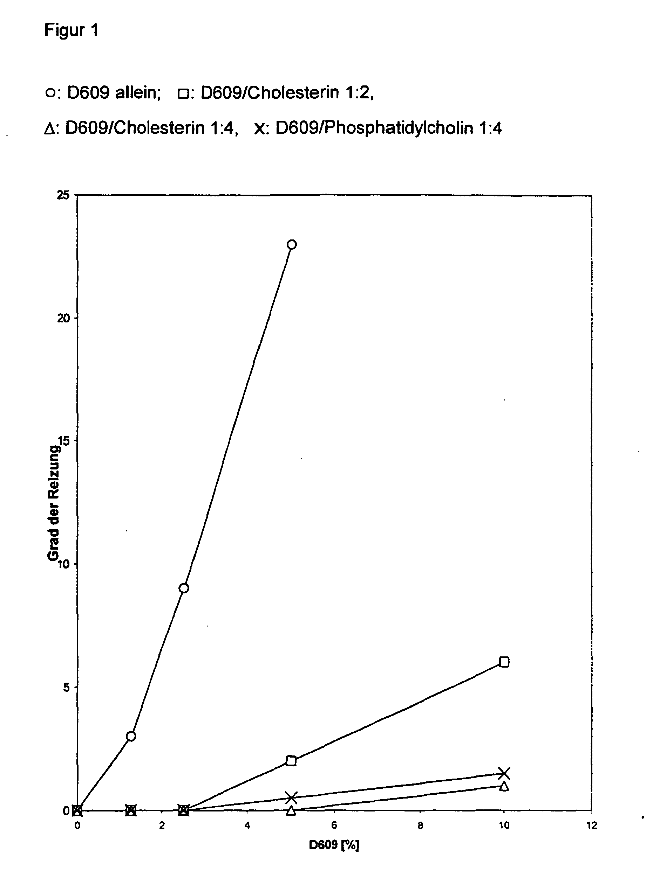

Je 10 haarlosen Mäusen (Stamm Swiss CD3, Nu/Nu) wurden jeweils 100 mg Salbe in der Nackenregion im Abstand von vier Stunden zwei mal aufgetragen. 12 Stunden nach der letzten Applikation wurde der Grad der Reizung abgelesen. Die Reizung wurde nach folgendem Schema bewertet: leichte Rötung 0,5; starke Rötung 1,0; Ulzerierung 2,0. Die Bewertungen aller Tiere einer Gruppe wurde addiert und in die Graphik (Figur 1) eingetragen. Wie man sieht, tritt bei einer Konzentration von 2,5 % D609 in reiner Vaseline bereits eine deutliche Reizung ein. Bei 5 % ist diese sehr stark ausgeprägt. Salben, die zwei Gewichtsteile Cholesterin enthielten zeigten eine deutliche Minderung der Symptome. Eine signifikante Reizung trat erst bei 10 % D609 ein. Salben, die vier Gewichtsteile Cholesterin enthielten, waren selbst bei einer Konzentration von 10 % D609 fast frei von Reizwirkung. Nur bei einem Tier wurde eine leichte Rötung beobachtet. Salben mit vier Gewichtsteilen Phosphatidylcholin wiesen ebenfalls eine deutliche Verbesserung der Verträglichkeit auf. Bei 5 % D609 zeigte ein Tier eine leichte Rötung, bei 10 % D609 fünf Tiere.

### Beispiel 4

### Antivirale Wirkung von D609 in Anwesenheit von Cholesterin.

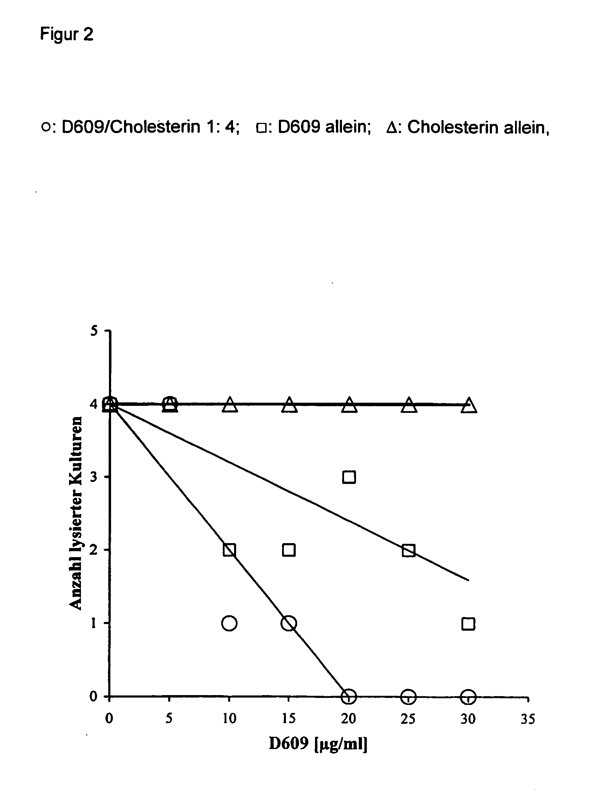

Affennierenzellen (Linie Rita) wurden in Linbroplatten mit 20 plaquebildenden Einheiten Herpes Simplex Virus 1 (HSV 1), Stamm Angelotti infiziert. Nach 15 min wurde frisches Zellkulturmedium (durch Reduktion der Natriumbikarbonatmenge auf 0,85 g /I und Zugabe von 1 ml 1 N HCl auf pH 6,8 eingestellt) das entweder 0, 5, 10, 15, 20, 25 oder 30 µg/ml D609 enthielt, zugegeben. Alle Ansätze wurden vierfach ausgeführt. Gleichzeitig wurden Kulturen mit D609 und der vierfachen Konzentration Cholesterin oder mit Cholesterin allein behandelt. Nach 5 Tagen waren in allen unbehandelten Kulturen die Affennierenzellen durch den zytopathogenen Effekt (cpe) lysiert. Nach weiteren drei Tagen wurde die Zahl der Kulturen mit cpe für jede D609 Konzentration bestimmt. Wie man aus Figur 2 entnehmen kann, nimmt die Zahl der Kulturen mit cpe mit steigender D609-Dosis ab. Die antivirale Wirkung von D609 wurde durch Cholesterin gesteigert. Während ohne Cholesterin 25 µg/ml D609 notwendig waren, um die Zahl der infizierten Kulturen um 50 % zu reduzieren, waren in Anwesenheit von Cholesterin nur 10 µg/ml D609 notwendig. Cholesterin allein hatte keinen Effekt auf die Vermehrung von HSV. Bis zur höchsten Konzentration (120 µg/ml) waren alle Kulturen durch HSV 1 lysiert. Die antivirale Wirkung von D609 wird unter Bedingungen, wie sie auf der Haut herrschen (pH 6,8) demnach um mehr als einen Faktor 2,5 verbessert.

### Beispiel 5

### Stabilität von D609 in wässriger Lösung in Anwesenheit von Cholesterin.

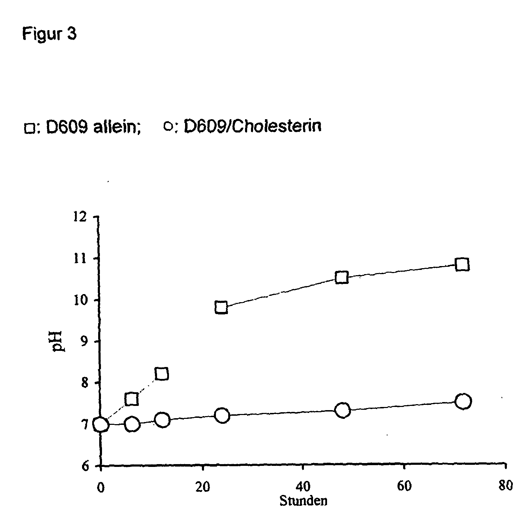

D609 wurde in einer Konzentration von 1 mg/ml in destilliertem Wasser gelöst. Jeweils 100 ml dieser Lösung wurden in 200 ml Erlenmeyerkolben gefüllt. Eine von zwei Lösungen wurde mit 1 g Cholesterin versetzt. Beide Lösungen wurden bei 37°C inkubiert. In regelmäßigen Abständen wurde der pH Wert der Lösungen bestimmt. D609 zerfällt in Anwesenheit von Wasser zu Tricyclodecanol, Schwefelkohlenstoff und KOH. Mit der Bildung von KOH steigt der pH Wert der Lösung. Der Anstieg des pH ist deshalb ein Maß für den Zerfall von D609. Aus Figur 3 ist ersichtlich, dass D609 in wässriger Lösung bereits innerhalb von 6 Stunden deutlichen Zerfall zeigt. In Anwesenheit von Cholesterin ist selbst nach 72 Stunden nur geringer pH-Anstieg und damit Zerfall zu beobachten.

Dieses Beispiel zeigt, dass eine sogar in wässriger Lösung durch einfaches Vermischen des Xanthogenats mit dem Emulgator eine Stabilisierung erreicht wird. Es ist keine Ultraschallbehandlung nötig.

## Patentansprüche

1. Arzneimittel zur Behandlung von Virus-, Tumor- oder Autoimmunerkrankungen, umfassend eine wasserfreie, pharmazeutische Formulierung, enthaltend ein Xanthogenat der Formel I 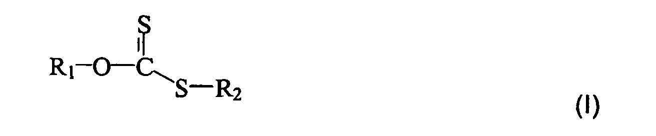 wobei R₁ für einen gegebenenfalls substituierten Aryl- oder Alkylrest steht und R₂ für ein Metallatom, eine gegebenenfalls substituierte Alkyl-, Alkoxy-, Amino- oder Ammoniumgruppe oder Halogen steht,
und einen Emulgator, ausgewählt unter Cholesterin, Lecithin, Phosphatidylserin, Sphingomyelin und Phosphatidylcholin,
sowie ein die Aktivität des Xanthogenats erhöhendes Adjuvans, ausgewählt unter ionischen Detergentien, Deoxycholinsäure oder deren pharmazeutisch verträglichen Salzen und Phosphonsäure, **dadurch gekennzeichnet, dass** es herstellbar ist, indem das Xanthogenat und das Adjuvans in Substanz mit dem Emulgator gemischt werden.

2. Mittel nach Anspruch 1, **dadurch gekennzeichnet, dass** R₁ ein Cyclododecyl-, Dodecyl-, Undecyl-, Decyl-, Tricyclo[5,2,1,0^{2,6}]-decyl-, nonyl-, octyl-, Bicyclo[2,2,1]-heptyl-, Cyclohexyl-, Hexyl- oder Toluoylrest ist.

3. Mittel gemäß Anspruch 1, **dadurch gekennzeichnet, dass** R₁ ein exo/exo-Tricyclo[5,2,1,0^{2,6}]-decylrest ist.

4. Mittel nach einem der Ansprüche 1 bis 3, **dadurch gekennzeichnet, dass** R₂ ein Natrium- oder Kaliumatom oder eine Dimethylglycylester- oder Methylestergruppe ist.

5. Mittel nach einem der Ansprüche 1 bis 4, **dadurch gekennzeichnet, dass** je ein Teil Xanthogenat 1 bis 10, vorzugsweise 2 bis 6 und insbesondere 4 Teile Emulgator enthalten sind.

6. Mittel nach Anspruch 1, **dadurch gekennzeichnet, dass** es Tricyclo[5,2,1,0^{2,6}]-decan-9-yl-xanthogenat als Xanthogenat, Cholesterin oder Phosphatidylcholin als Emulgator und das Natrium- oder Kaliumsalz der Decansäure als Adjuvans enthält.

7. Mittel nach einem der Ansprüche 1 bis 6, **dadurch gekennzeichnet, dass** es eine Salbe ist, die die pharmazeutische Formulierung in einer lipophilen Substanz, vorzugsweise Vaseline, enthält.

8. Mittel nach einem der Ansprüche 1 bis 6, **dadurch gekennzeichnet, dass** es als Kit, enthaltend die Formulierung als wasserfreie Zubereitung und ein physiologisch verträgliches Medium zur Herstellung einer Injektionslösung, bereitgestellt wird.

9. Mittel nach Anspruch 8, **dadurch gekennzeichnet, dass** die pharmazeutische Formulierung oder das physiologisch verträgliche Medium ein Alkalihydroxid, insbesondere KOH, enthält.

## Claims

1. Medicament for the treatment of viral diseases, oncoses or autoimmune diseases comprising an anhydrous, pharmaceutical formulation, comprising a xanthate of the formula I 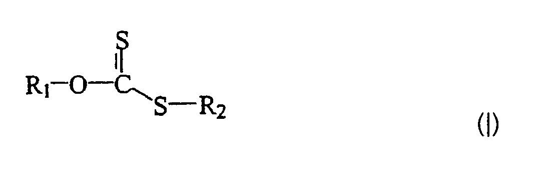 where R₁ is an optionally substituted aryl or alkyl radical and
R₂ is a metal atom, an optionally substituted alkyl, alkoxy, amino or ammonium group or halogen,
and an emulsifier which is selected from cholesterol, lecithin, phosphatidyl-serine, sphingomyelin and phosphatidylcholine
and an adjuvant increasing the activity of the xanthate chosen from an ionic detergent, deoxycholic acid or a pharmaceutically tolerable salt thereof, and a phosphonic acid
**characterized in that**
obtainable by mixing the xanthate and the adjuvant with the emulsifier in substance.

2. Medicament as claimed in claim 1, **characterized in that** R₁ is a cyclododecyl, dodecyl, undecyl, decyl, tricyclo[5,2,1,0^{2,6}]decyl, nonyl, octyl, bicyclo [2,2,1] heptyl, cyclohexyl, hexyl or toluoyl radical.

3. Medicament as claimed in claim 1, **characterized in that** R₁ is an exo/exo-tricyclo[5,2,1,0^{2,6}]decyl radical.

4. Medicament according to any one of claims 1 to 3, **characterized in that** R₂ is a sodium or potassium atom or a dimethylglycyl ester or methyl ester group.

5. Medicament according to any one of claims 1 to 4, **characterized in that** 1 to 10, preferably 2 to 6, and in particular 4, parts of emulsifier are contained per one part of xanthate.

6. Medicament according to claim 1, **characterized in that** it contains tricyclo-[5,2,1,0^{2,6}]decan-9-yl xanthate as the xanthate, cholesterol or phosphatidylcholine as the emulsifier and the sodium or potassium salt of decanoic acid as the adjuvant.

7. Medicament according to any one of claims 1 to 6, **characterized in that** it is an ointment which contains the pharmaceutical formulation in a lipophilic substance, preferably petroleum jelly.

8. Medicament according to any one of claims 1 to 6, **characterized in that** it is made available as a kit containing the formulation in the form of an anhydrous composition and a physiologically tolerable medium for preparing an injection solution.

9. Medicament according to claim 8, **characterized in that** the pharmaceutical formulation or the physiologically tolerable medium contains an alkali metal hydroxide, in particular KOH.

## Revendications

1. Médicament pour le traitement de maladies virales, tumorales ou auto-immunes, comprenant une formulation anhydre, pharmaceutique, contenant un xanthogénate de formule I 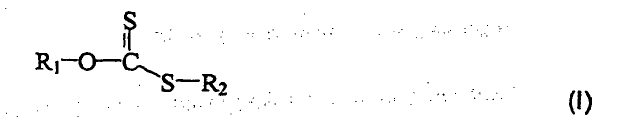 dans laquelle R₁ représente un radical aryle ou alkyle le cas échéant substitué et R₂ représente un atome métallique, un groupe alkyle, alcoxy, amino ou ammonium le cas échéant substitué ou halogène,
et un émulsifiant, choisi parmi le cholestérol, la lécithine, la phosphatidylsérine, la sphingomyéline et la phosphatidylcholine,
ainsi qu'un adjuvant augmentant l'activité du xanthogénate, choisi parmi les détergents ioniques, l'acide désoxycholique ou ses sels pharmaceutiquement acceptables et l'acide phosphonique, **caractérisé en ce qu'**il peut être préparé **en ce que** le xanthogénate et l'adjuvant sont mélangés en masse avec l'émulsifiant.

2. Médicament selon la revendication 1, **caractérisé en ce que** R₁ est un radical cyclododécyle, dodécyle, undécyle, décyle, tricyclo[5,2, 1,0^{2,6}]-décyle, nonyle, octyle, bicyclo[2,2,1]-heptyle, cyclohexyle, hexyle ou tolyle.

3. Médicament selon la revendication 1, **caractérisé en ce que** R₁ est un radical exo/exo-tricyclo[5,2,1,0^{2,6}]-décyle.

4. Médicament selon l'une quelconque des revendications 1 à 3, **caractérisé en ce que** R₂ est un atome de sodium ou de potassium ou un groupe ester de diméthylglycyle ou ester de méthyle.

5. Médicament selon l'une quelconque des revendications 1 à 4, **caractérisé en ce qu'**il contient par partie de xanthogénate 1 à 10, de préférence 2 à 6 et en particulier 4 parties d'émulsifiant.

6. Médicament selon la revendication 1, **caractérisé en ce qu'**il contient du xanthogénate de tricyclo[5,2,1,0^{2,6}]-décan-9-yle comme xanthogénate, du cholestérol ou de la phosphatidylcholine comme émulsifiant et le sel sodique ou potassique de l'acide décanoïque comme adjuvant.

7. Médicament selon l'une quelconque des revendications 1 à 6, **caractérisé en ce qu'**il s'agit d'une pommade, qui contient la formulation pharmaceutique dans une substance lipophile, de préférence la vaseline.

8. Médicament selon l'une quelconque des revendications 1 à 6, **caractérisé en ce qu'**il est mis à disposition sous forme d'un kit, contenant la formulation sous forme de composition anhydre et un milieu physiologiquement acceptable pour la préparation d'une solution destinée à une injection.

9. Médicament selon la revendication 8, **caractérisé en ce que** la formulation pharmaceutique ou le milieu physiologiquement compatible contient un hydroxyde de métal alcalin, en particulier le KOH.
